# EUROPEAN PATENT APPLICATION

(11) **EP 1 731 170 A1**
(43) Date of publication of application: **13.12.2006**
(21) Application number: 05726976.3
(22) Date of filing: 25.03.2005
(51) Int. Cl.: A61K 45/00, A61K 31/192, A61K 31/421, A61K 31/426, A61K 31/4427, A61K 31/517, A61P 3/10

(54) **THERAPEUTIC AGENT FOR DIABETES CONTAINING INSULIN RESISTANCE IMPROVING AGENT**

(30) Priority: 29.03.2004 JP 2004094598
(71) Applicant: Sankyo Company, Limited, Tokyo 103-8426 (JP)
(72) Inventor: KANDA, Shoichi, c/o Sankyo Company, Limited, Shinagawa-ku, Tokyo 140-8710 (JP); ARAKI, Kazushi, c/o Sankyo Company, Limited, Shinagawa-ku, Tokyo 140-8710 (JP); OHSUMI, Jun, c/o Sankyo Company, Limited, Shinagawa-ku, Tokyo 140-8710 (JP)
(74) Representative: Gibson, Christian John Robert
(86) International application number: PCT/JP2005/005526
(87) International publication number: WO 2005/092382

(57) **Abstract**

An object of the present invention is to provide a highly safe method for treating a disease, in which side effects (for example, edema) are suppressed while maintaining excellent pharmaceutical effects. There is provided a pharmaceutical composition including an insulin sensitizer as an active ingredient, **characterized in that** a cycle of administration of the insulin sensitizer wherein the dosage thereof is reduced or withdrawn during the administration period is repeated once or more.

## Description

### Technical Field

The present invention relates to a pharmaceutical composition comprising an insulin sensitizer which enables the dosage and side effects thereof to be reduced while maintaining a high efficacy, and a method for administering the same.

### Background Art

Insulin sensitizers are used as therapeutic agents for diabetes mellitus because they have excellent antihyperglycemic activity. Insulin sensitizers currently on the market include pioglitazone and rosiglitazone, pioglitazone being administered once daily,and rosiglitazone once to twice daily, to diabetic patients.

However, it is known that insulin sensitizers sometimes cause adverse events such as heart failure, edema, and hydrothorax, and it is therefore considered necessary that adequate caution is exercised in their use. Currently known methods for inhibiting or treating such adverse events include, as one example, combination with other agents (see, for example, Patent Document 1).

The duration of administration of agents for treating diabetes mellitus extends over long periods of time particularly due to its nature as a chronic metabolic disease. Accordingly, there is a current need for such an agent which has high safety and for which burdens on patients (such as, for example, an economic burden and a burden of frequency of ingestion) are reduced.

[Patent Document 1]: Japanese Patent Laid-Open No. 2002-255854 (International Publication WO 02/051441 pamphlet)

### Disclosure of the Invention

The present inventors have carried out intensive studies for the purpose of developing a highly safe method for treating a disease (particularly, diabetes mellitus) which exerts excellent effect and in which side effects (such as, for example, edema) can be suppressed. As a result, the inventors have found that the side effects of an insulin sensitizer can be significantly suppressed while maintaining the efficacy thereof by performing the administration cycle that: (1) an effective dose of the agent is administered and (2) the dosage thereof is then significantly reduced to a low dose or withdrawn, thereby accomplishing the present invention.

The present invention provides:
(1) A pharmaceutical composition comprising an insulin sensitizer as an active ingredient, characterized in that, in a method for treating a disease, a cycle of administration of the insulin sensitizer wherein the dosage thereof is reduced or withdrawn during the administration period is repeated once or more;
(2) A pharmaceutical composition comprising an insulin sensitizer as an active ingredient, characterized in that, in a method for treating a disease, the dosage of the insulin sensitizer is reduced during the administration period;
(3) A pharmaceutical composition comprising an insulin sensitizer as an active ingredient, characterized in that, in a method for treating a disease, the dosage of the insulin sensitizer is reduced from the initial dosage by a factor of 10 to 100 during the administration period;
(4) A pharmaceutical composition comprising an insulin sensitizer as an active ingredient, characterized in that, in a method for treating a disease, the administration of the insulin sensitizer is changed from daily administration to one- to three-day-a-week administration for use;
(5) The pharmaceutical composition described in any of items (1) to (4) above, wherein the pharmaceutical composition comprising an insulin sensitizer as an active ingredient is a pharmaceutical composition for the prophylaxis or treatment of diabetes mellitus;
(6) The pharmaceutical composition described in any of items (1) to (4) above, wherein the pharmaceutical composition comprising an insulin sensitizer as an active ingredient is a pharmaceutical composition for the prophylaxis or treatment of impaired glucose tolerance (IGT);
(7) The pharmaceutical composition described in any of items (1) to (4) above, wherein the pharmaceutical composition comprising an effective dose of an insulin sensitizer as an active ingredient is a pharmaceutical composition for the prophylaxis or treatment of diseases associated with insulin resistance;
(8) A pharmaceutical composition comprising a low or lower dose of an insulin sensitizer as an active ingredient, for administration to a subject to be treated having their blood glucose level controlled by the ingestion of a medicine comprising the insulin sensitizer as an active ingredient;
(9) The pharmaceutical composition described in any of items (1) to (8) above, wherein the insulin sensitizer is a PPARγ agonist;
(10) The pharmaceutical composition described in any of items (1) to (8) above, wherein the insulin sensitizer is a thiazolidinedione insulin sensitizer;
(11) The pharmaceutical composition described in any of items (1) to (8) above, wherein the insulin sensitizer ispioglitazone, rosiglitazone, MCC-555, NN-2344, BMS-298585, AZ-242, LY-519818, or TAK-559 represented by the following formulae: 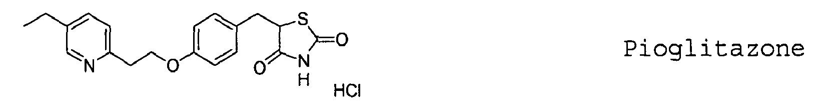 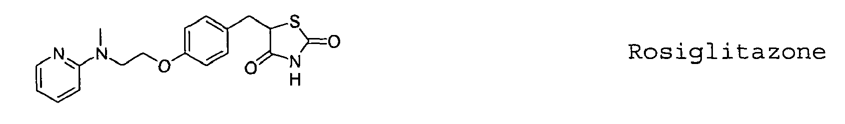 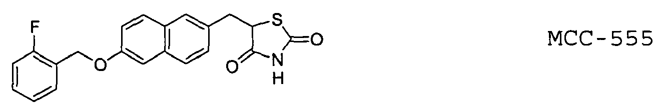 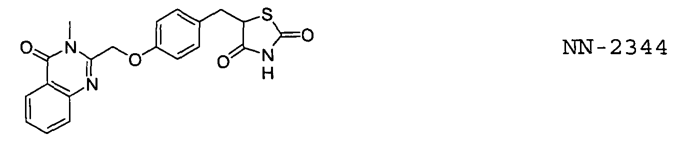 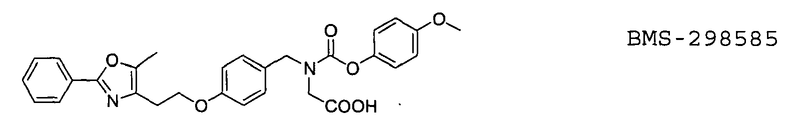 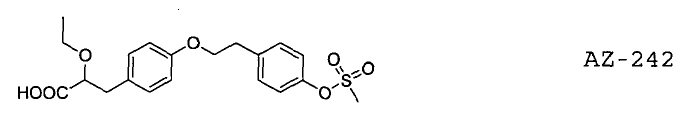 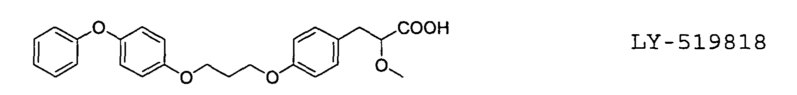 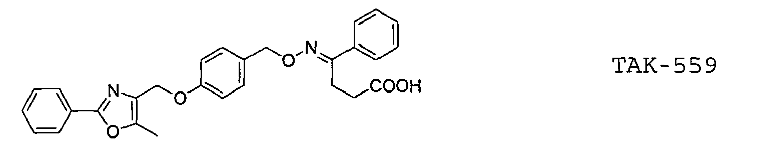 , 3-(2,4-dichlorobenzyl)-2-methyl-N-(pentylsulfonyl)-3H-benzimidazole-5-carboxamide (FK-614), 5-[4-(6-methoxy-1-methyl-1H-benzimidazol-2-ylmethoxy)ben zyl]thiazolidin-2,4-dione, or a pharmacologically acceptable salt thereof;
(12) The pharmaceutical composition described in any of items (1) to (8) above, wherein the insulin sensitizer ispioglitazone, rosiglitazone, MCC-555, NN-2344, BMS-298585, AZ-242, LY-519818, or TAK-559 represented by the following formulae: 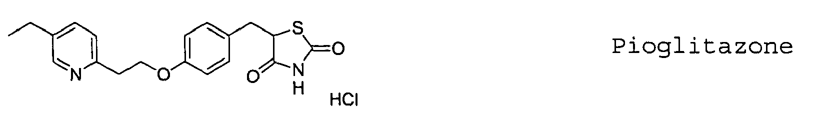 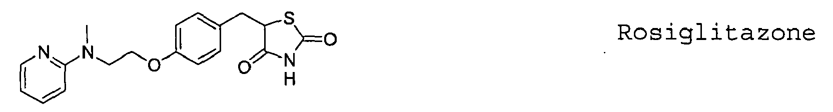 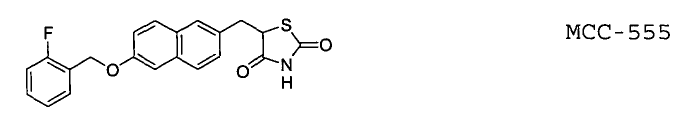 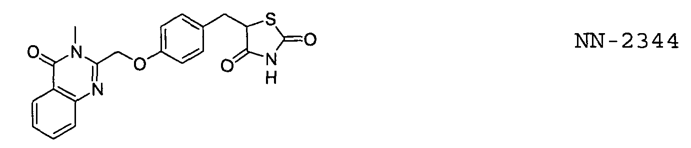 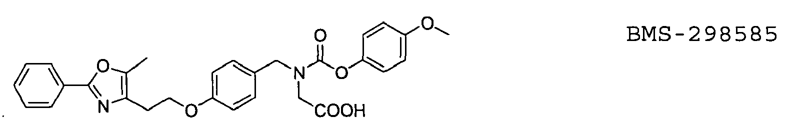 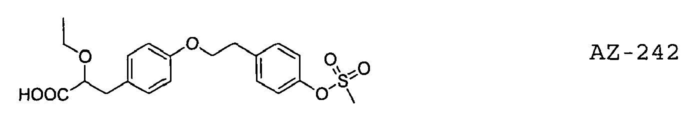 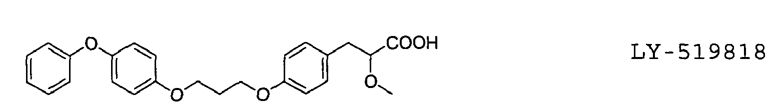 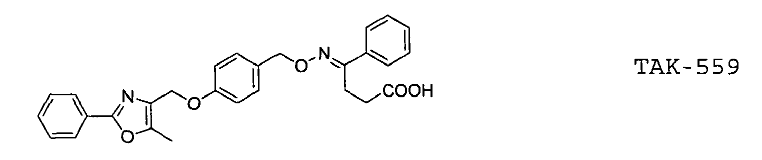 5-[4-(6-methoxy-1-methyl-1H-benzimidazol-2-ylmethoxy)ben zyl]thiazolidin-2,4-dione, or a pharmacologically acceptable salt thereof;
(13) The pharmaceutical composition described in any of items (1) to (8) above, wherein the insulin sensitizer is pioglitazone or rosiglitazone represented by the following formulae: 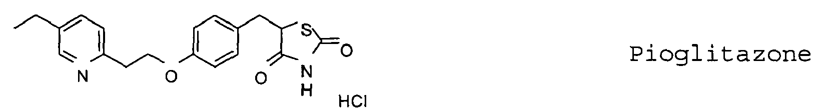 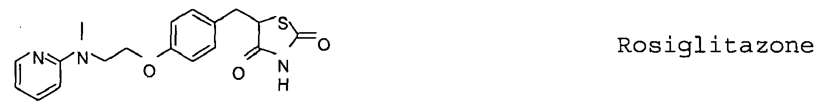 , 5-[4-(6-methoxy-1-methyl-1H-benzimidazol-2-ylmethoxy)ben zyl]thiazolidin-2,4-dione, or a pharmacologically acceptable salt thereof;
(14) The pharmaceutical composition described in any of items (1) to (8) above, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof;
(15) A therapeutic agent for the treatment of diabetes mellitus comprising an insulin sensitizer as an active ingredient, characterized in that, in a method for treating diabetes mellitus, the dosage of the insulin sensitizer is reduced during the administration period;
(16) A therapeutic agent for the treatment of diabetes mellitus comprising an insulin sensitizer as an active ingredient, wherein a method for treating diabetes mellitus comprises the steps of (a) administering to a subject to be treated the insulin sensitizer at a dose effective for the subj ect to be treated and then (b) administering to the subj ect the insulin sensitizer at a low dose, thereby achieving a therapeutic effect for diabetes mellitus while preventing a side effect in the subject to be treated;
(17) The therapeutic agent for the treatment of diabetes mellitus described in item (16) above, wherein the side effect is caused by the insulin sensitizer;
(18) The therapeutic agent for the treatment of diabetes mellitus described in item (16) above, wherein the side effect is hemodilution caused by the insulin sensitizer;
(19) The therapeutic agent for the treatment of diabetes mellitus described in any of items (15) to (18) above, wherein the insulin sensitizer is a PPARγ agonist;
(20) The therapeutic agent for the treatment of diabetes mellitus described in any of items (15) to (18) above, wherein the insulin sensitizer is a thiazolidinedione insulin sensitizer;
(21) The therapeutic agent for the treatment of diabetes mellitus described in any of items (15) to (18) above, wherein the insulin sensitizer is pioglitazone, rosiglitazone, MCC-555, NN-2344, BMS-298585, AZ-242, LY-519818, or TAK-559 represented by the following formulae: 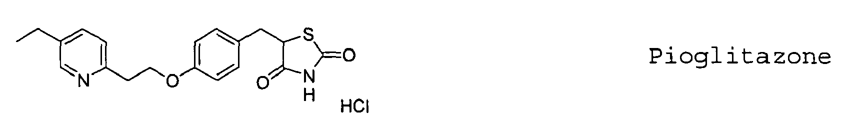 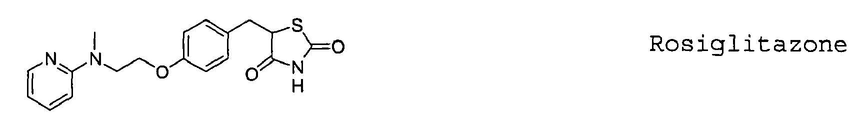 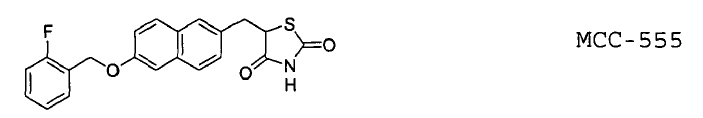 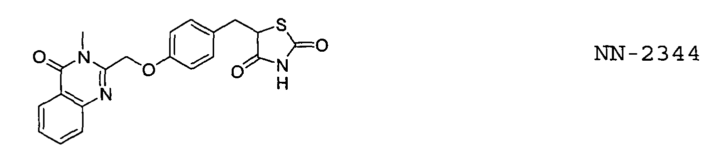 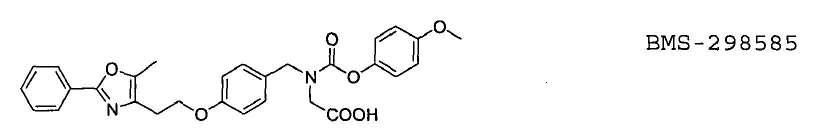 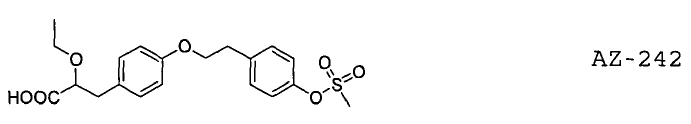 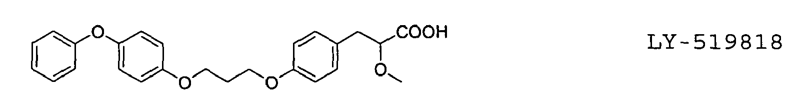 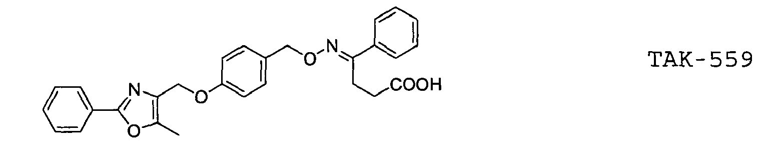 , 3-(2,4-dichlorobenzyl)-2-methyl-N-(pentylsulfonyl)-3H-benzimidazole-5-carboxamide (FK-614), 5-[4-(6-methoxy-1-methyl-1H-benzimidazol-2-ylmethoxy)ben zyl]thiazolidin-2,4-dione, or a pharmacologically acceptable salt thereof;
(22) The therapeutic agent for the treatment of diabetes mellitus described in any of items (15) to (18) above, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof;
(23) Use of an insulin sensitizer for the manufacture of a medicine characterized in that, in treating diabetes mellitus, the insulin sensitizer as an active ingredient is administered at a reduced dose during the administration period;
(24) Use of an insulin sensitizer for the manufacture of a medicine comprising a low or lower dose of the insulin sensitizer as an active ingredient for administration to a subject to be treated having their blood glucose level controlled by the ingestion of a medicine comprising the insulin sensitizer as an active ingredient;
(25) The use described in item (23) or (24) above, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof;
(26) A kit comprising an agent, the kit comprising (a) a medicine comprising an effective dose of an insulin sensitizer and (b) a medicine comprising a low dose of the insulin sensitizer, for using the medicine comprising a low dose of the insulin sensitizer after sufficiently improving symptoms by employing the medicine comprising an effective dose of the insulin sensitizer;
(27) A kit for treating diabetes mellitus comprising a medicine for treating diabetes mellitus, the kit comprising (a) a medicine comprising an effective dose of an insulin sensitizer and (b) a medicine comprising a low dose of the insulin sensitizer, for using the medicine comprising a low dose of the insulin sensitizer after sufficiently lowering the blood glucose level by employing the medicine comprising an effective dose of the insulin sensitizer;
(28) The kit for treating diabetes mellitus described in item (26) or (27) above, wherein the insulin sensitizer is 5-[4-(6-methoxy-1-methyl-1H-benzimidazol-2-ylmethoxy)ben zyl]thiazolidin-2,4-dione or a pharmacologically acceptable salt thereof;
(29) Use of an insulin sensitizer for the manufacture of a kit comprising a medicine for treating a disease, the kit comprising (a) a medicine comprising an effective dose of an insulin sensitizer and (b) a medicine comprising a low dose of the insulin sensitizer, for using the medicine comprising a low dose of the insulin sensitizer after sufficiently improving symptoms by employing the medicine comprising an effective dose of the insulin sensitizer;
(30) Use of an insulin sensitizer for the manufacture of a kit comprising a medicine for treating diabetes mellitus, the kit comprising (a) a medicine comprising an effective dose of an insulin sensitizer and (b) a medicine comprising a low dose of the insulin sensitizer, for using the medicine comprising a low dose of the insulin sensitizer after sufficiently lowering the blood glucose level by employing the medicine comprising an effective dose of the insulin sensitizer;
(31) The use described in item (29) or (30) above, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof;
(32) A method for preparing an agent for treating a disease, characterized in that a medicine comprising an effective dose of an insulin sensitizer and a medicine comprising a low dose of the insulin sensitizer are used;
(33) A method for preparing an agent for treating diabetes mellitus, characterized in that a medicine comprising an effective dose of an insulin sensitizer and a medicine comprising a low dose of the insulin sensitizer are used;
(34) The method for preparing an agent described in item (32) or (33) above, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof;
(35) A method for treating a disease, characterized in that the method comprises repeating once or more the cycle of (a) the administration of an effective dose of an insulin sensitizer to a subject to be treated and (b) the following administration of a low dose of the insulin sensitizer to the subject or the withdrawal thereof is repeated once or more;
(36) A method for treating a disease, characterized in that the method comprises administering an effective dose of an insulin sensitizer to a subject to be treated and then administering a low dose of the insulin sensitizer thereto;
(37) A method for treating diabetes mellitus, characterized in that the method comprises administering an effective dose of an insulin sensitizer to a subject to be treated and then administering a low dose of the insulin sensitizer thereto;
(38) A method for treating diabetes mellitus, characterized in that the method comprises administering an effective dose of an insulin sensitizer to a subject to be treated and then administering a low dose of the insulin sensitizer thereto to alleviate a side effect attributable to the insulin sensitizer; and
(39) The treatment method described in any of items (35) to (38) above, wherein the insulin sensitizer is 5-[4-(6-methoxy-1-methyl-1H-benzimidazol-2-ylmethoxy)ben zyl]thiazolidin-2,4-dione or a pharmacologically acceptable salt thereof.

In the present invention, "effective dose" refers generally to a dose of agent considered to be effective for treating a disease of interest, or a dose higher than that. By way of example, for diabetes mellitus, it is a dose of agent judged by a physician to be effective for blood glucose control after a certain period of drug administration (preferably, 3 months) to an individual, and thus may vary depending on the age, sex, and symptoms of the individual. Of the effective doses, the minimum dose (a dose lower than which is considered to be ineffective) represents the "minimum effective dose" for the individual.

In the present invention, "low dose" refers to a dose lower than the minimum effective dose.

In the present invention, "insulin sensitizer" is not particularly restrictedprovided that it is an agent improving insulin resistance and augmenting insulin sensitivity, but examples thereof include pioglitazone, rosiglitazone, MCC-555, NN-2344, BMS-298585, AZ-242, LY-519818, andTAK-559 represented by the following formulae: 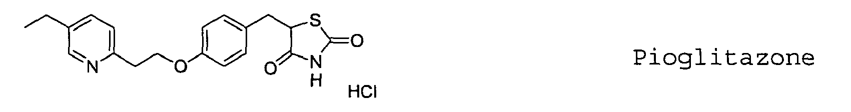 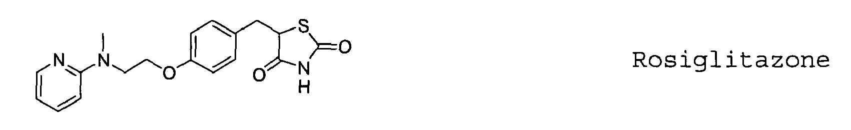 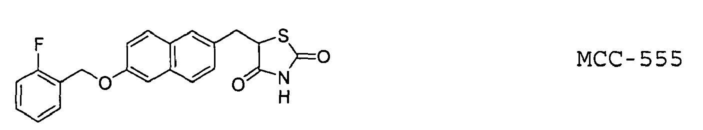 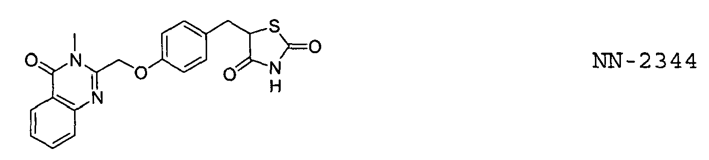 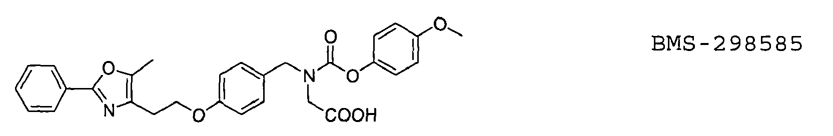 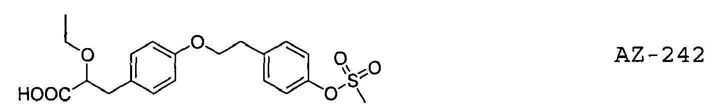 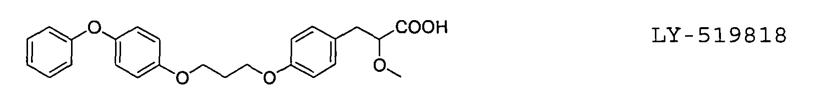 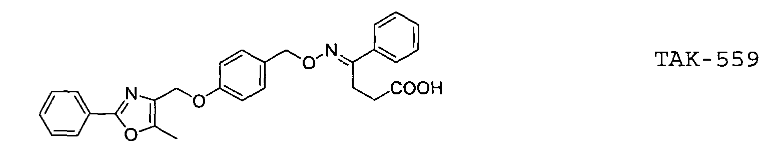 , 3-(2,4-dichlorobenzyl)-2-methyl-N-(pentylsulfonyl)-3H-benzimidazole-5-carboxamide (FK-614), 5-[4-(6-methoxy-1-methyl-1H-benzimidazol-2-ylmethoxy)ben zyl]thiazolidin-2,4-dione, and pharmacologically acceptable salts thereof. Preferred are thiazolidinedione insulin sensitizers such as pioglitazone, rosiglitazone, 5-[4-(6-methoxy-1-methyl-1H-benzimidazol-2-ylmethoxy)ben zyl]thiazolidin-2,4-dione, and pharmacologically acceptable salts thereof.

Pioglitazone is a compound described in U.S. Patent No. 4,687,777. Rosiglitazone is a compound described in U.S. Patent No. 5, 002, 953. MCC-555 is a compound described in U. S. Patent No. 5,594,016. NN-2344 is a compound described in International Publication WO 97/41097 pamphlet. BMS-298585 is a compound described in International Publication WO 01/21602 pamphlet. AZ-242 is a compound described in International Publication WO 99/62872 pamphlet. LY-519818 is a compound described in International Publication WO 02/100813 pamphlet. TAK-559 is a compound described in Japanese Patent Laid-Open No. 2000-34266. 3-(2,4-Dichlorobenzyl)-2-methyl-N-(pentylsulfonyl)-3H-benzimidazole-5-carboxamide (FK-614) is a compound described in U.S. Patent No. 6,166,219. 5-[4-(6-Methoxy-1-methyl-1H-benzimidazol-2-ylmethoxy)ben zyl] thiazolidin-2,4-dione and a salt thereof can be produced according to methods described in Japanese Patent Laid-Open No. 09-295970, EP 0745600, U.S. Patent No. 5,886,014, and International Publication WO 00/71540 pamphlet.

In the present invention, "PPARγ agonist" is not particularly restricted provided that it is an agent activating a peroxisome proliferator-activated receptor (PPAR)γ, and is known to have therapeutic effects against diabetes mellitus and/or cancer as effects of action thereof. Preferred examples thereof include compounds having a thiazolidinedione skeleton such as the above-described insulin sensitizers.

In the present invention, "disease associated with insulin resistance" is not particularly restricted provided that it is a disease induced by an aggravation in insulin resistance, but examples thereof include diabetes mellitus, hyperglycemia, impaired glucose tolerance, obesity, hyperlipemia, diabetic complications, gestational diabetes mellitus, and polycystic ovarian disorder. Preferred are diabetes mellitus, hyperglycemia, and impaired glucose tolerance.

The route of administration of the insulin sensitizer used in the present invention is typically an oral route. The unit dosage form thereof is not particularly restricted provided that it is prepared by a conventional preparation technique, but examples thereof include powders, granules, tablets, and capsules.

These various preparations can be formulated by a conventional method using known auxiliary agents generally usable in the field of medicinal preparation, such as an excipient, binder, disintegrant, lubricant, solubilizer, flavouring agent, and coating agent.

In forming into a tablet, for example, those heretofore well-known as carriers in this field can be widely used, and examples thereof can include excipients such as lactose, saccharose, sodium chloride, dextrose, urea, starch, calcium carbonate, kaolin, crystalline cellulose, and silicic acid, binders such as water, ethanol, propanol, simple syrup, dextrose in water, starch in water, gelatin solution, carboxymethyl cellulose, shellac, methyl cellulose, potassium phosphate, and polyvinylpyrrolidone, disintegrants such as dry starch, sodium alginate, powdered agar, powdered laminaran, sodium bicarbonate, calcium carbonate, polyoxyethylene sorbitan fatty acid esters, sodium lauryl sulfate, monoglyceride stearate, starch, and lactose, disintegration inhibitors such as saccharose, stearin, cocoa butter, and hydrogenated oil, absorption promoters such as quaternary ammonium base and sodium lauryl sulfate, humectants such as glycerin and starch, adsorbents such as starch, lactose, kaolin, bentonite, and colloidal silicic acid, and lubricants such as purified talc, stearate, powdered boric acid, and polyethyleneglycol. In addition, such tablets may optionally be conventional coated tablets such as sugar-coated tablets, gelatin-coated tablets, enteric-coated tablets, and film coated tablets, or double-layer tablets or multilayer tablets.

In forming into a pill, those heretofore known as carriers in this field can be widely used, and examples thereof can include excipients such as dextrose, lactose, starch, cocoa butter, hydrogenated vegetable oil, kaolin, and talc, binders such as powdered acacia, powdered tragacanth, gelatin, and ethanol, and disintegrants such as laminaran agar. In addition, the pill may optionally contain a colourant, a preservative, a perfume, a seasoning agent, a sweetening agent, or other medicines.

The amount of active ingredient compound contained in any of the above-described medicinal preparations is not particularly restricted and is properly selected in wide range, but it is appropriate that the amount thereof is typically 1 to 70 wt.%, preferably 1 to 30 wt.% based on the total composition.

In the present invention, the insulin sensitizer is administered once or in several divided portions in a daily dose corresponding to the effective dose or low dose of the agent used, or given in an effective dose at least one day apart.

In the present invention, the side effects (e.g. , edema, cardiac hypertrophy, body fluid retention, and hydrothorax) of an insulin sensitizer can be suppressed while maintaining the excellent pharmaceutical effects (particularly, hypoglycemic effect) thereof by performing the administration cycle that: (1) an effective dose of the agent is administered and (2) the dosage thereof is then significantly reduced to a low dose or withdrawn. Thus, the insulin sensitizer and method for administering the same according to the present invention are useful for treating diseases associated with insulin resistance (particularly, diabetes mellitus).

### Brief Description of the Drawings

Figure 1 is a graph showing the time course of blood glucose level during the administration of the effective and low doses of compoundAas an insulin sensitizer in combination (Example 1);
Figure 2 is a graph showing the time course of red blood cell count during the administration of the effective and low doses of compound A as an insulin sensitizer in combination (Example 1);
Figure 3 is a graph showing the comparison of heart weight between the respective groups during the administration of the effective and low doses of compound A as an insulin sensitizer in combination (Example 1);
Figure 4 is a graph showing the time course of blood glucose level during the intermittent administration of compound A as an insulin sensitizer (Example 2);
Figure 5 is a graph showing the time course of red blood cell count during the intermittent administration of compound A as an insulin sensitizer (Example 2); and
Figure 6 is a graph showing the comparison of heart weight between the respective groups during the intermittent administration of compound A as an insulin sensitizer (Example 2).

### Best Mode for Carrying Out the Invention

Then, the present invention is described in further detail with reference to the Examples; however, it is not intended to be limited thereto.

### Examples

Compound A used in the Examples is a compound stated in methods described in Japanese Patent Laid-Open No. 09-295970, EP 0745600, U.S. Patent No. 5,886,014, and International Publication WO 00/71540 pamphlet, and can be produced according to the methods of these publications.

### Example 1

The effect of alleviating side effects resulting from the administration of the effective and low doses of 5-[4-(6-methoxy-1-methyl-1H-benzimidazol-2-ylmethoxy)ben zyl]thiazolidin-2,4-dione hydrochloride (compound A) in combination.

Zucker diabetic fatty rats (9-week-old, from Charles River Laboratories Japan, Inc.) were used in the experiment. The rats were divided into 4 groups of 5 individuals each so that body weight, blood glucose level, and red blood cell count may each be uniform among the groups, and each of these groups served as a low-dose treatment group (Ia), a low-dose treatment group (Ib), a continuous treatment group, or a control group. Food (FR2, from Funabashi Noj o) and water were given ad libitum during the testing period.

A 0.5% carboxymethyl cellulose (CMC) solution only was administered to the control group, and a CMC suspension of compound A was orally administered to all of the other groups, once a day in doses described below. For the low-dose treatment group (Ia), it was administered at 3 mg/kg for 2 weeks and then at a decreased dose of 0.03 mg/kg for a further 3 weeks. For the low-dose treatment group (Ib), it was administered at 3 mg/kg for 2 weeks and then at a decreased dose of 0.3 mg/kg for a further 3 weeks. For the continuous treatment group, 3 mg/kg was administered for 5 weeks.

Blood was collected every 7 days from the start of administration using a capillary tube treated with heparin and EDTA, and blood glucose level and blood cell parameters were determined using an autoanalyzer (blood glucose level: Glucoroder GX-T fromA&T Corporation), blood cell parameters: KX-21N from Sysmex Corporation).

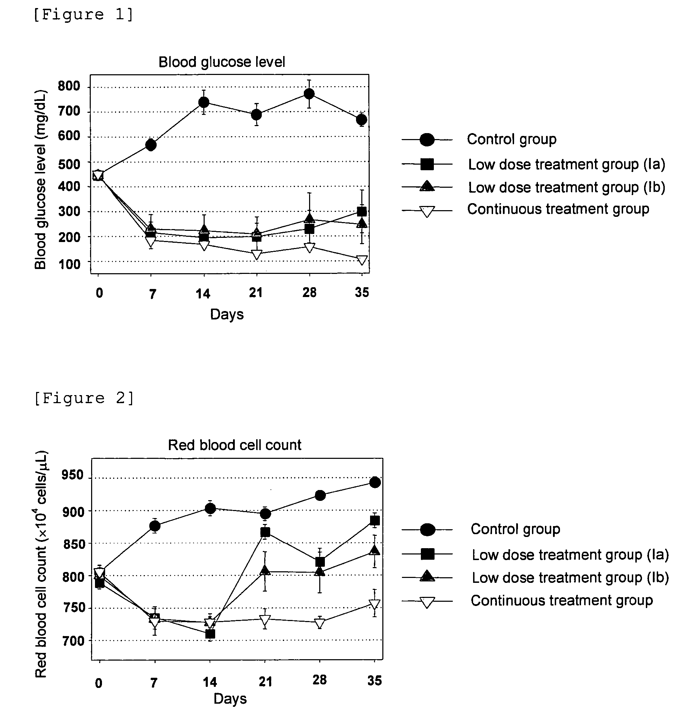

The time course of blood glucose level of each group is shown in Figure 1. As shown in this figure, in the continuous treatment group, the blood glucose level was lowered to a normal value, and the state then continued. The administration of the effective dose of compound A rapidly lowered the blood glucose level also in the low-dose treatment group (Ia) and low-dose treatment group (Ib). In addition, in the low-dose treatment group (Ia) and low-dose treatment group (Ib), the blood glucose level was depressed to 300 mg/dL or less as a mean even after the decrease of compound A to the low doses (from the 14th day on). In other words, it is indicated that the blood glucose level is highly favourably controlled even when compound A is decreased to a dose much lower than the usual dose thereof.

The time course of red blood cell count of each group is shown in Figure 2. From this figure, hemodilution (a decrease in red blood count) probably due to an increase in plasma volume was observed in the continuous treatment group. Incontrast, it was rapidly recovered in dose-dependent manner after decreasing compound A in the low-dose treatment group (Ia) and low-dose treatment group (Ib), and recovered nearly to control level in the low-dose treatment group (Ia) . These results show that the reduced dose of compound A markedly improved the hemodilution.

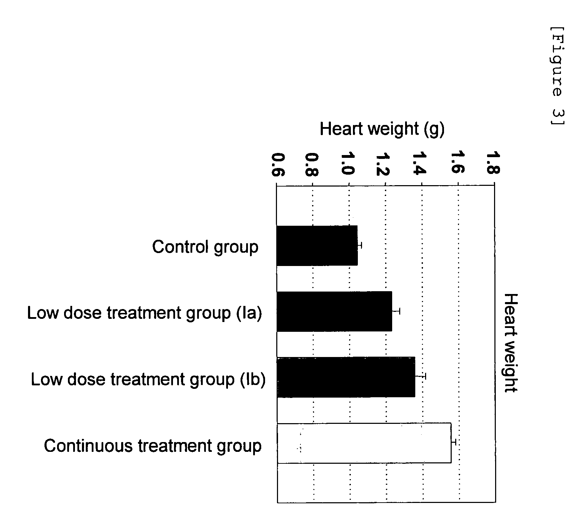

Figure 3 shows heart weights in the respective groups. From this figure, a marked increase in heart weight associated with a long-term increase in plasma volume was observed in the continuous treatment group. On the other hand, heart weights in the low-dose treatment group (Ia) and low-dose treatment group (Ib) increased depending on the dose of compound A, but were clearly decreased values compared to that in the continuous treatment group. These results show that the reduced dose of compound A markedly suppressed the increase in heart weight.

### Example 2

The effect of alleviating side effects resulting from the intermittent administration of 5-[4-(6-methoxy-1-methyl-1H-benzimidazol-2-ylmethoxy)ben zyl]thiazolidin-2,4-dione hydrochloride (compound A).

Zucker diabetic fatty rats (9-week-old, from Charles River Laboratories Japan, Inc.) were used in the experiment. The rats were divided into 3 groups so that body weight, blood glucose level, and red blood cell count may each be uniform among the groups, and each of these groups served as a control group, an intermittent treatment group, or a continuous treatment group (n = 4 to 5). Food (FR2, from Funabashi Nojo) and water were given ad libitum during the testing period.

A 0.5% carboxymethyl cellulose (CMC) solution only was administered to the control group, and a CMC suspension of compound A was forcibly orally administered to the intermittent treatment group and continuous treatment group according to the regimen described below. For the intermittent treatment group, compound A was repeatedly given in a dose of 1 mg/kg once a day for one week before decreasing the administration thereof to twice a week to administer it for a further two weeks (dosing days: days 0 to 7, 10, 14, and 17). In this respect, the CMC solution was given on the days when compound A was not administered. For the continuous treatment group, 1 mg/kg was repeatedly administered once a day for three weeks.

Blood was collected at days 0, 2, 5, 7, 9, 12, 14, 16, 19, and 21 when the start date of dosing was shown as day 0, using a capillary tube treated with heparin, and blood glucose level and blood cell parameters were determined using an autoanalyzer (blood glucose level: Glucoroder GX-T from A&T Corporation, blood cell parameters: KX-21N from Sysmex Corporation).

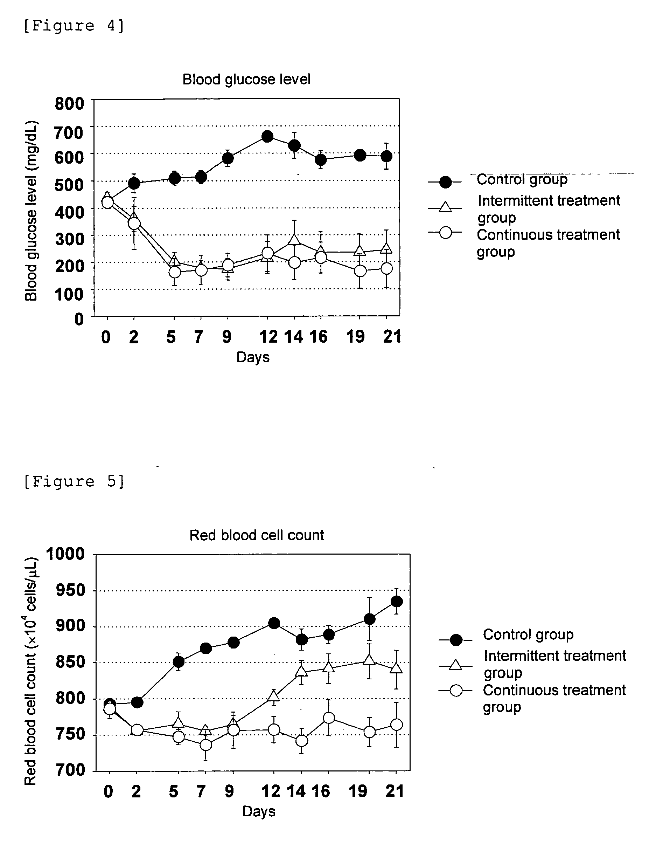

The time course of blood glucose level of each group is shown in Figure 4. As shown in this figure, in the continuous treatment group, the blood glucose level was lowered to a normal range, and the state then continued. The one-week repeated administration of compound A rapidly lowered the blood glucose level also in the intermittent treatment group. Then, decreasing the administration of compound A to twice a week kept the blood glucose level at nearly the same level as that in the continuous treatment group. In other words, it is indicated that the blood glucose level is favourably controlled even when withdrawal/administration of compound A is repeated.

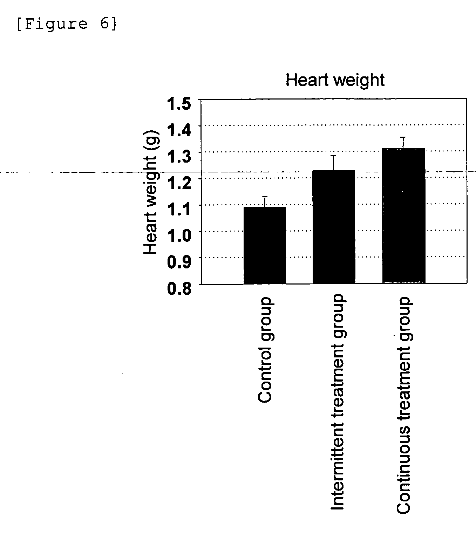

The time course of red blood cell count of each group is shown in Figure 5. From this figure, hemodilution (a decrease in red blood cell count) probably due to an increase in plasma volume was observed in the continuous treatment group. In contrast, in the intermittent treatment group, an increase in red blood cell count was observed when the administration of compound A was decreased to twice a week. These results show that repeated withdrawal/administration of compound A improved the hemodilution.

Figure 6 shows heart weights in the respective groups. From this figure, a marked increase in heart weight associated with a long-term increase in plasma volume was observed in the continuous treatment group. On the other hand, heart weight in the intermittent treatment group was decreased in value compared to that in the continuous treatment group. These results show that repeated withdrawal/administration of compound A suppressed the increase in heart weight.

Summarizing the above-described results, it was demonstrated that administering an effective dose of an insulin sensitizer to improve insulin resistance before reducing the dose can specifically alleviate the side effects (e.g., decreased red blood cell count and increased heart weight) thereof while maintaining good control of blood glucose level. In addition, administering an effective dose of an insulin sensitizer before repeating withdrawal and administration thereof can also produce similar effects.

Possible causes of the effect that only such side effects are suppressed include that once insulin resistance is greatly improved by the administration of an effective dose of an insulin sensitizer, the improved state is persistent and good glycemic control therefore continues even after the decrease or withdrawal of the agent. Alternatively, because side effects such as increased plasma volume are induced in proportion to the dose of an insulin sensitizer per se, the reduction of the dose or the withdrawal of the administration probably produced the rapid, dose-dependent recovery of red blood cell count and the reduction of an increase in heart weight.

From the above-described results, the side effects of an insulin sensitizer can be alleviated while favourably controlling blood glucose level by carrying out the administration cycle that: (1) an effective dose thereof is administered and (2) the dosage thereof is then significantly reduced to a low dose or withdrawn (in the case of the withdrawal, the administration is preferably performed one to three days a week, more preferably two days a week), which can thus probably provide a highly excellent method for administering the insulin sensitizer. Further, as long as an insulin sensitizer can be safely used for a long period of time, the necessity of additionally ingesting other agents for the prophylaxis or treatment of adverse events is eliminated, which, together with a reduction in the dose, will be highly effective for greatly lightening burdens on patients.

## Claims

1. A pharmaceutical composition comprising an insulin sensitizer as an active ingredient, **characterized in that**, in a method for treating a disease, a cycle of administration of the insulin sensitizer wherein the dosage thereof is reduced or withdrawn during the administration period is repeated once or more.

2. A pharmaceutical composition comprising an insulin sensitizer as an active ingredient, **characterized in that**, in a method for treating a disease, the dosage of the insulin sensitizer is reduced during the administration period.

3. A pharmaceutical composition comprising an insulin sensitizer as an active ingredient, **characterized in that**, in a method for treating a disease, the dosage of the insulin sensitizer is reduced from the initial dosage by a factor of 10 to 100 during the administration period.

4. A pharmaceutical composition comprising an insulin sensitizer as an active ingredient, **characterized in that**, in a method for treating a disease, the administration of the insulin sensitizer is changed from daily administration to one- to three-day-a-week administration.

5. The pharmaceutical composition according to any of claims 1 to 4, wherein the pharmaceutical composition comprising an insulin sensitizer as an active ingredient is a pharmaceutical composition for the prophylaxis or treatment of diabetes mellitus.

6. The pharmaceutical composition according to any of claims 1 to 4, wherein the pharmaceutical composition comprising an insulin sensitizer as an active ingredient is a pharmaceutical composition for the prophylaxis or treatment of impaired glucose tolerance (IGT).

7. The pharmaceutical composition according to any of claims 1 to 4, wherein the pharmaceutical composition comprising an effective dose of an insulin sensitizer as an active ingredient is a pharmaceutical composition for the prophylaxis or treatment of diseases associated with insulin resistance.

8. A pharmaceutical composition comprising a low or lower dose of an insulin sensitizer as an active ingredient, for administration to a subject to be treated having their blood glucose level controlled by the ingestion of a medicine comprising the insulin sensitizer as an active ingredient.

9. The pharmaceutical composition according to any of claims 1 to 8, wherein the insulin sensitizer is a PPARγ agonist.

10. The pharmaceutical composition according to any of claims 1 to 8, wherein the insulin sensitizer is a thiazolidinedione insulin sensitizer.

11. The pharmaceutical composition according to any of claims 1 to 8, wherein the insulin sensitizer is pioglitazone, rosiglitazone, MCC-555, NN-2344, BMS-298585, AZ-242, LY-519818, or TAK-559 represented by the following formulae: 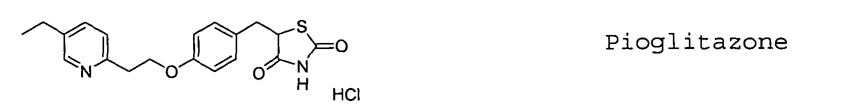 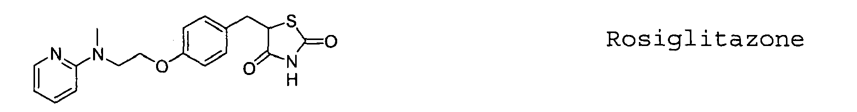 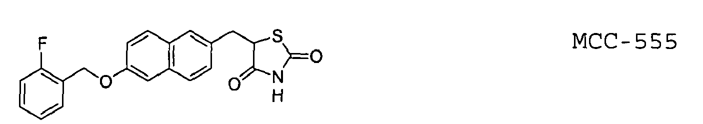 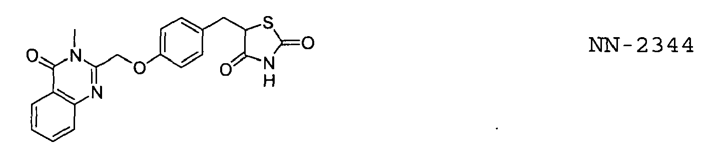 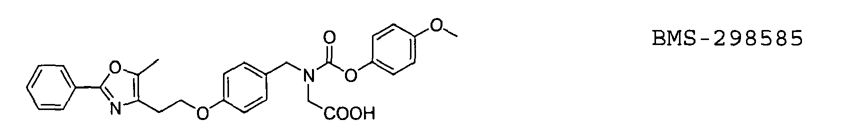 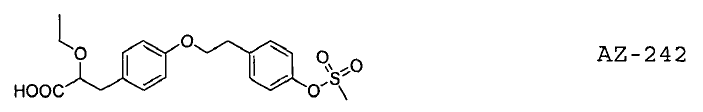 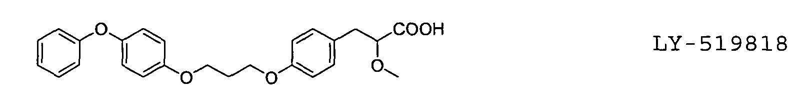 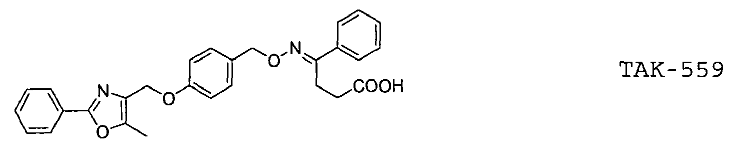 , 3-(2,4-dichlorobenzyl)-2-methyl-N-(pentylsulfonyl)-3H-benzimidazole-5-carboxamide (FK-614), 5-[4-(6-methoxy-1-methyl-1H-benzimidazol-2-ylmethoxy)ben zyl]thiazolidin-2,4-dione, or a pharmacologically acceptable salt thereof.

12. The pharmaceutical composition according to any of claims 1 to 8, wherein the insulin sensitizer is pioglitazone, rosiglitazone, MCC-555, NN-2344, BMS-298585, AZ-242, LY-519818, or TAK-559 represented by the following formulae: 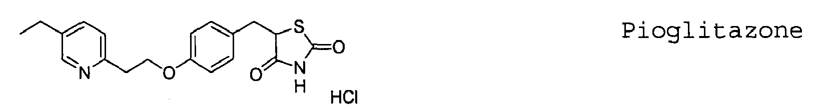 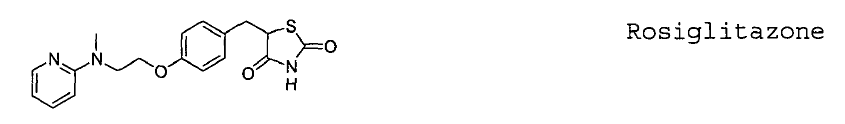 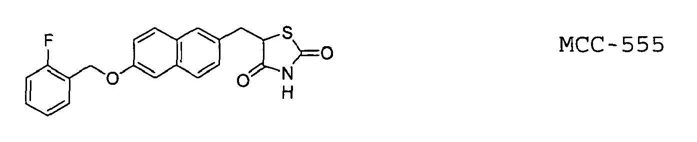 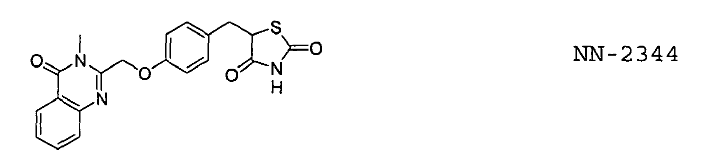 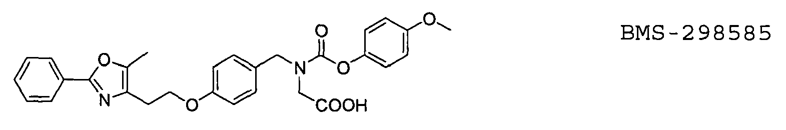 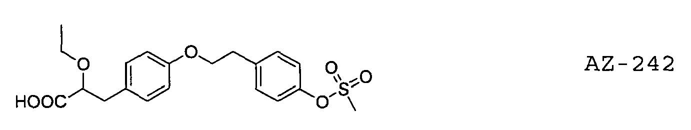 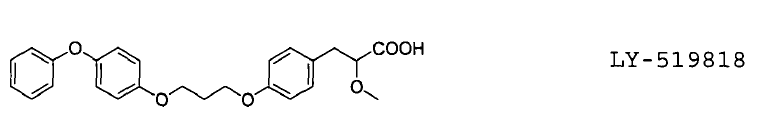 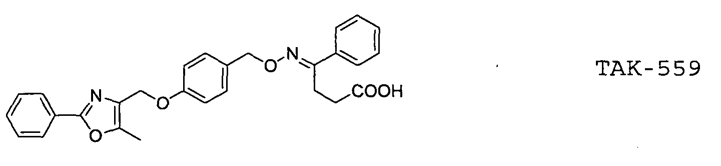 , 5-[4-(6-methoxy-1-methyl-1H-benzimidazol-2-ylmethoxy)ben zyl]thiazolidin-2,4-dione, or a pharmacologically acceptable salt thereof.

13. The pharmaceutical composition according to any of claims 1 to 8, wherein the insulin sensitizer is pioglitazone or rosiglitazone represented by the following formulae: 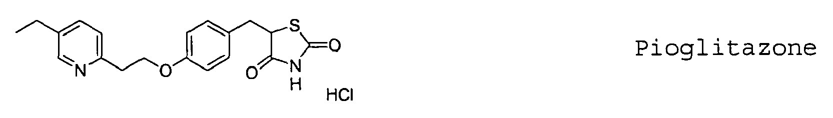 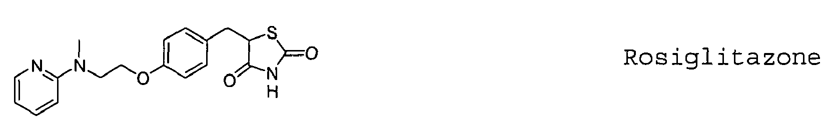 , 5-[4-(6-methoxy-1-methyl-1H-benzimidazol-2-ylmethoxy)ben zyl]thiazolidin-2,4-dione, or a pharmacologically acceptable salt thereof.

14. The pharmaceutical composition according to any of claims 1 to 8, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof.

15. A therapeutic agent for the treatment of diabetes mellitus comprising an insulin sensitizer as an active ingredient, **characterized in that**, in a method for treating diabetes mellitus, the dosage of the insulin sensitizer is reduced during the administration period.

16. A therapeutic agent for the treatment of diabetes mellitus comprising an insulin sensitizer as an active ingredient, wherein a method for treating diabetes mellitus comprises the steps of (a) administering to a subject to be treated the insulin sensitizer at a dose effective for the subj ect to be treated and then (b) administering to the subj ect the insulin sensitizer at a low dose, thereby achieving a therapeutic effect for diabetes mellitus while preventing a side effect in the subject to be treated.

17. The therapeutic agent for the treatment of diabetes mellitus according to claim 16, wherein the side effect is caused by the insulin sensitizer.

18. The therapeutic agent for the treatment of diabetes mellitus according to claim 16, wherein the side effect is hemodilution caused by the insulin sensitizer.

19. The therapeutic agent for the treatment of diabetes mellitus according to any of claims 15 to 18, wherein the insulin sensitizer is a PPARγ agonist.

20. The therapeutic agent for the treatment of diabetes mellitus according to any of claims 15 to 18, wherein the insulin sensitizer is a thiazolidinedione insulin sensitizer.

21. The therapeutic agent for the treatment of diabetes mellitus according to any of claims 15 to 18, wherein the insulin sensitizer is pioglitazone, rosiglitazone, MCC-555, NN-2344, BMS-298585, AZ-242, LY-519818, or TAK-559 represented by the following formulae: 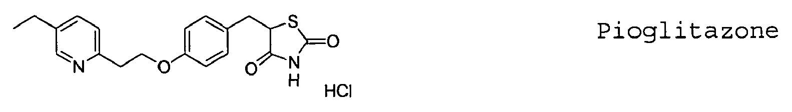 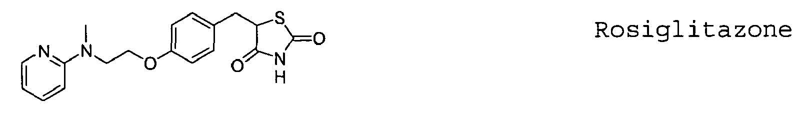 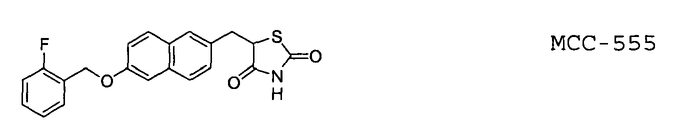 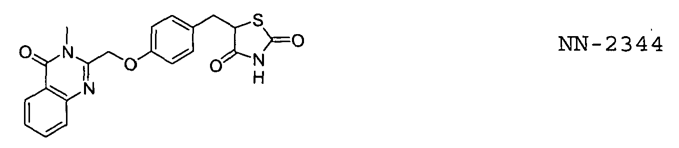 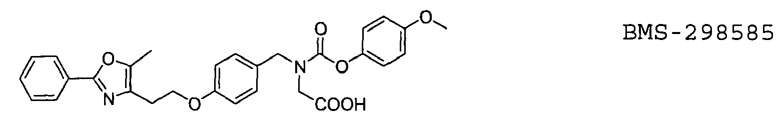 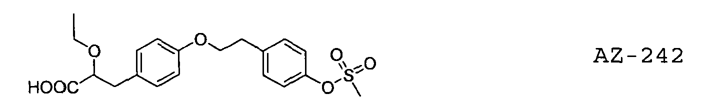 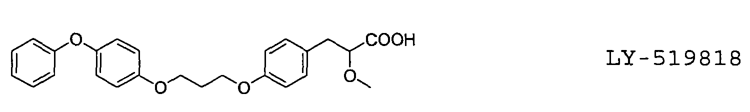 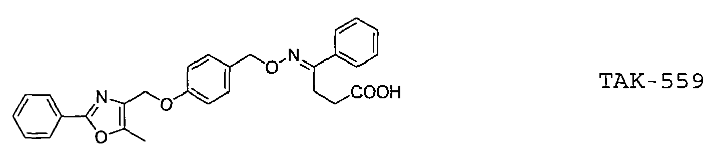 , 3-(2,4-dichlorobenzyl)-2-methyl-N-(pentylsulfonyl)-3H-benzimidazole-5-carboxamide (FK-614), 5-[4-(6-methoxy-1-methyl-1H-benzimidazol-2-ylmethoxy)ben zyl]thiazolidin-2,4-dione, or a pharmacologically acceptable salt thereof.

22. The therapeutic agent for the treatment of diabetes mellitus according to any of claims 15 to 18, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof.

23. Use of an insulin sensitizer for the manufacture of a medicine **characterized in that**, in treating diabetes mellitus, the insulin sensitizer as an active ingredient is administered at a reduced dose during the administration period.

24. Use of an insulin sensitizer for the manufacture of a medicine comprising a low or lower dose of the insulin sensitizer as an active ingredient for administration to a subject to be treated having their blood glucose level controlled by the ingestion of a medicine comprising the insulin sensitizer as an active ingredient.

25. The use according to claim 23 or 24, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof.

26. A kit comprising an agent, the kit comprising (a) a medicine comprising an effective dose of an insulin sensitizer and (b) a medicine comprising a low dose of the insulin sensitizer, for using the medicine comprising a low dose of the insulin sensitizer after sufficiently improving symptoms by employing the medicine comprising an effective dose of the insulin sensitizer.

27. A kit for treating diabetes mellitus comprising a medicine for treating diabetes mellitus, the kit comprising (a) a medicine comprising an effective dose of an insulin sensitizer and (b) a medicine comprising a low dose of the insulin sensitizer, for using the medicine comprising a low dose of the insulin sensitizer after sufficiently lowering the blood glucose level by employing the medicine comprising an effective dose of the insulin sensitizer.

28. The kit for treating diabetes mellitus according to claim 26 or 27, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof.

29. Use of an insulin sensitizer for the manufacture of a kit comprising a medicine for treating a disease, the kit comprising (a) a medicine comprising an effective dose of an insulin sensitizer and (b) a medicine comprising a low dose of the insulin sensitizer, for using the medicine comprising a low dose of the insulin sensitizer after sufficiently improving symptoms by employing the medicine comprising an effective dose of the insulin sensitizer.

30. Use of an insulin sensitizer for the manufacture of a kit comprising a medicine for treating diabetes mellitus, the kit comprising (a) a medicine comprising an effective dose of an insulin sensitizer and (b) a medicine comprising a low dose of the insulin sensitizer, for using the medicine comprising a low dose of the insulin sensitizer after sufficiently lowering the blood glucose level by employing the medicine comprising an effective dose of the insulin sensitizer.

31. The use according to claim 29 or 30, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof.

32. A method for preparing an agent for treating a disease, **characterized in that** a medicine comprising an effective dose of an insulin sensitizer and a medicine comprising a low dose of the insulin sensitizer are used.

33. A method for preparing an agent for treating diabetes mellitus, **characterized in that** a medicine comprising an effective dose of an insulin sensitizer and a medicine comprising a low dose of the insulin sensitizer are used.

34. The method for preparing an agent according to claim 32 or 33, wherein the insulin sensitizer is 5-[4-(6-methoxy-1-methyl-1H-benzimidazol-2-ylmethoxy)ben zyl]thiazolidin-2,4-dione or a pharmacologically acceptable salt thereof.

35. A method for treating a disease, **characterized in that** the method comprises repeating once or more the cycle of (a) the administration of an effective dose of an insulin sensitizer to a subject to be treated and (b) the following administration of a low dose of the insulin sensitizer to the subject or the withdrawal thereof.

36. A method for treating a disease, **characterized in that** the method comprises administering an effective dose of an insulin sensitizer to a subject to be treated and then administering a low dose of the insulin sensitizer thereto.

37. A method for treating diabetes mellitus, **characterized in that** the method comprises administering an effective dose of an insulin sensitizer to a subject to be treated and then administering a low dose of the insulin sensitizer thereto.

38. A method for treating diabetes mellitus, **characterized in that** the method comprises administering an effective dose of an insulin sensitizer to a subject to be treated and then administering a low dose of the insulin sensitizer thereto to alleviate a side effect attributable to the insulin sensitizer.

39. The treatment method according to any of claims 35 to 38, wherein the insulin sensitizer is 5-[4-(6-methoxy-1-methyl-1H-benzimidazol-2-ylmethoxy)ben zyl] thiazolidin-2,4-dione or a pharmacologically acceptable salt thereof.
